Europäisches Patentamt

(19) European Patent Office

Office européen des brevets

(11) Publication number: **0 319 074 B1**

(12) **EUROPEAN PATENT SPECIFICATION**

(45) Date of publication of patent specification :
**29.01.92 Bulletin 92/05**

(21) Application number : **88202621.4**

(22) Date of filing : **22.11.88**

(51) Int. Cl.⁵ : **A61K 31/65,** A61K 9/20

(54) **Pharmaceutical composition and process for its preparation.**

The file contains technical information
submitted after the application was filed and
not included in this specification

(30) Priority : **30.11.87 EP 87202370**

(43) Date of publication of application :
**07.06.89 Bulletin 89/23**

(45) Publication of the grant of the patent :
**29.01.92 Bulletin 92/05**

(84) Designated Contracting States :
**AT BE CH DE ES FR GB GR IT LI LU NL SE**

(56) References cited :
**EP-A- 0 159 735**
**EP-A- 0 281 200**
**GB-A- 2 058 565**
**GB-A- 2 172 006**

(56) References cited :
**DIE PHARMAZIE, vol. 33, no. 11, November
1978, pages 747-749, VEB Verlag Volk und
Gesundheit, Berlin, DE; G. NAGY et al.:
"Untersuchungen über die Textur und die
Eigenschaften von Acetylsalicylsäure-Tablet-
ten"
J. Pharm. Pharmac. Vol. 28, 1976, pages
234-238**

(73) Proprietor : **GIST-BROCADES N.V.
Wateringseweg 1
NL-2611 XT Delft (NL)**

(72) Inventor : **Akkerboom, Piet Johannes
Dr. J.W. Paltelaan 48
NL-2712 RT Zoetermeer (NL)**
Inventor : **De Cocq, Robert
Wielewaalstraat 16
NL-2623 GT Delft (NL)**
Inventor : **Wegman, Bernardus Bartholomeus
Maria
Norholm 58
NL-2133 HL Hoofddorp (NL)**

(74) Representative : **Huygens, Arthur Victor, Dr. et
al
c/o Gist-Brocades N.V. Patent & Trademarks
Department Wateringseweg 1 PO Box 1
NL-2600 MA Delft (NL)**

## Description

The invention relates to a pharmaceutical composition and more particularly to a tablet containing a tetracycline, which exhibits a rapid disintegration when immersed in water, to granulates for making such tablets and to processes for their preparation.

It is generally accepted that the therapeutic action of a medicine in a living organism depends to a considerable extent on the carrier in which the medicine is administered. For drugs which are taken orally, especially high demands are made upon the pharmaceutical preparation.

The first demand is a high bioavailability : as much active substance as possible should be made available to the organism. In the treatment of infections with an antibiotic-containing composition, which is the object of this invention, optimal levels of antibiotic in the blood should be reached in the shortest possible time.

A second demand is that the dosage form should be free from administration problems. However, the dosage form with the best bioavailability is seldom easy to use and on the other hand, one which is easy to use often does not have a satisfactory bioavailability.

Tablets are pharmaceutical formulations with several advantages : they are easy to use with respect to dosing, storing, carrying about and administering. However, many patients experience difficulties when swallowing a tablet and moreover the absorption may be contingent on its dissolution rate.

An aqueous solution or dispersion of the drug is easily ingested by the patient, is better absorbed and frequently gives high initial blood concentrations. However, such a liquid preparation is cumbersome to dose and to carry about. Frequently it is best prepared shortly before delivery in the pharmacy and should be stored at low temperature. Sometimes preserving agents need to be added to such a liquid preparation.

Therefore, so-called disperse tablets have been developed which, when immersed in water, rapidly disintegrate to a suspension of the drug, thus combining certain advantages of both dry and liquid dosage forms. Tablets of this type are described in European patent application EP-A-0181650, German patent DE 1617343 and in J. Pharm. Pharmac. (1976), 28, 234-238. The latter publication describes granulates containing a tetracycline and microcrystalline cellulose and prepared by wet granulation without a wet granulation binder. However, none the granulates, which all contain less than 7.75 wt% microcrystalline cellulose, shows a disintegration time which is less than 2.8 minutes. Known disperse tablets are not completely satisfactory, however, especially when directly ingested, because either the tablets disintegrate already in the mouth to form a pappy mass which is unpleasant to swallow or the full disintegration takes considerably more than one minute. For some kinds of drugs, such as β-lactam antibiotics, improved types of disperse tablets have been developed, as described in European patent application EP-A-0281200. British patent application GB 2058565 describes a double layer penicillin tablet with pivampicillin in one layer and mecillinam hydrochloride in the other one. The tablet is said to be easily disintegratable, but any indication of a disintegration time is lacking. Tetracyclines, especially doxycycline, are much prescribed medicines. However, no process is yet known for the preparation of tablets containing a tetracycline, which can either be easily swallowed, or provide quickly a fine dispersion when immersed in water.

## SUMMARY OF THE INVENTION

It is therefore an object of this invention to provide a tablet containing a tetracycline which can be used in two different ways. When immersed in water it quickly disperses to provide an excellent, easy to ingest suspension. Alternatively the tablet can be easily swallowed as such.

A further object of the invention is to provide a simple pharmaceutical process for industrial scale preparation of the tablets. These aims are achieved by combining the tetracycline drug with certain adjuvants into a new composition, which permits the tablet to be prepared either using a wet granulation method or by direct compression.

## DETAILS OF THE INVENTION

The tablet according to this invention is one that contains at least a tetracycline, microcrystalline cellulose or microfine cellulose or a mixture of both, low-substituted hydroxypropylcellulose and a thickening agent.

Microcrystalline cellulose is the common name for purified, partially depolymerized cellulose occurring as a crystalline powder, composed of porous particles. It is a widely used adjuvant, known e.g. under the name AVICEL® (registered trademark). Microfine cellulose (e.g. ELCEMA® (registered trademark)), also denoted as powdered cellulose, is a mechanically processed a-cellulose from fibrous plant materials. It is a common pharmaceutical binder and disintegrant.

In this description "cellulose product" refers particularly to microcrystalline cellulose and microfine cellulose and to mixtures of them.

Microcrystalline cellulose or microfine cellulose is used in amounts of 20-60 wt%, preferably 35-50 wt% and particularly 45 wt%. These and all other percentages mentioned in this description and the appending claims are based on the weight of the tetracycline.

Low-substituted hydroxypropylcellulose acts as a disintegrant. With this name a common pharmaceutical excipient is denoted.

The thickening agent may be a natural gum, such as guar gum or gum arabic, or a cellulose derivative such as methylcellulose, ethylcellulose or hydroxyethylcellulose, but the preferred thickening agent is hydroxypropyl methylcellulose, an adjuvant which is available in various viscosity grades. The contribution of the thickening agent to the viscosity should be a low one. Therefore the viscosity of a 2% solution of the thickening agent in water, measured at 20°C, should be less than 50 mPa · s (cP) preferably less than 10 mPa · s (cP) and most preferably 5 mPa · s (cP).

The thickening agent acts in conjuction with low-substituted hydroxypropyl cellulose to provide the tablet with accurately timed disintegration behaviour. The tablet disintegrates at a rate which is sufficiently slow for swallowing it easily, but fast enough, when immersed in water, to give an excellent suspension within 60 seconds.

The low-substituted hydroxypropylcellulose and the thickening agent are used in a total amount of 10-40 wt%, preferably 20-30 wt% and are present in weight ratios of low-substituted hydroxypropylcellulose to thickening agent of from 3 : 1 to 10 : 1. Particularly good results are obtained with a weight ratio of 5 : 1.

Further conventional adjuvants, e.g. lubricants such as silica gel and magnesium stearate, flavours and fillers such as lactose and starch may also be added to the tablet composition.

The invention is particularly suitable for the formulation of doxycycline, especially in the form of a hydrate, in particular the monohydrate, or in any other form having a sufficiently low solubility to be virtually tasteless. Further suitable tetracyclines are, for example, tetracycline trihydrate, oxytetracycline dihydrate and the calcium salt of chlorotetracycline.

The tablet according to the invention may be prepared by the usual direct compression method. However, doxycycline monohydrate occurs in different crystal habits, some of which give rise to serious tabletting problems due to bad flow and compression properties of the tabletting mixture.

Therefore the invention further provides a tabletting method based on wet granulation, but using the ingredients of the direct compression recipe, which obviates this problem.

The present method comprises mixing the tetracycline with microcrystalline cellulose or microfine cellulose or a mixture of both, in amounts as suggested above, and adding 40-100 wt%, preferably 50-80 wt% of water, to give a wet mass, which is then granulated by methods known in the art. The granules are then dried to a moisture content of preferably less than 2 wt%, and passed through a sieve with small pores, preferably 0.71 mm. It should be noticed that the granulate is made without using a substantial amount, i.e. 0-0.5 wt% and preferably 0-0.1 wt% of the usual wet granulation binding substance, such as starch, sugars, polyvinyl pyrrolidone or cellulose esters. Surprisingly, a granulate of good quality is easily obtained.

The granulate is then mixed with low-substituted hydroxypropylcellulose and a thickening agent in amounts as suggested above to give a tabletable granulate of good flow, which, optionally with further adjuvants, can be easily compressed to tablets in the usual way.

The tablets according to the invention possess excellent properties. They can, at the patients choice, either be swallowed as such or used in aqueous suspension, since they disperse, when immersed in water, and often within 30-60 seconds, into a very fine suspension without leaving coarse granules.

The dispersion time is established (in duplo) by putting the tablet in a 100 ml glass containing 50 ml water of 20°C. After 30 s the contents are whirled until no lumps can be seen anymore. Time is read and the dispersion is poured immediately through a 0.71 mm sieve, which should not retain any particle. The shortest time in which this condition is fulfilled is the dispersion time.

With respect to the speed and the amount of absorption of the tetracycline into the blood, the tablet of the invention and the suspension made from it are equivalent.

It is also surprising that the tablet of the invention exhibits its useful properties irrespective whether it is prepared by direct compression or by the described wet granulation method. This feature contributes substantially to the flexibility of the manufacturing process.

The following examples will serve as illustrations.

Example 1

Doxycycline monohydrate (105.8 g) and microcrystalline cellulose (45 g) are mixed for 15 minutes in a planetary mixer. The mixture is then granulated with 60 ml of water. After 10 minutes of kneading, the resulting wet mass is passed through a 2 mm sieve and the wet granulation product dried at about 40°C until its water content is below 2% by weight. The granulate is then passed through a 0.71 mm sieve and is mixed for 20 minutes with lowsubstituted hydroxypropylcellulose LH11 (18 g), hydroxypropyl methylcellulose of 5 mPa · s (cP) viscosity (4 g), saccharin 10 g colloidal silica (0.6 g) and enough lactose to bring the total weight to 248 g. Then magnesium stearate (2 g) is added and the mixing continued for an additional 2 minutes. The resulting mixture is compressed into tablets each of about 250 mg, about 9 mm diameter and a hardness of 70-100 N or into tablets each of about 125 mg having a hardness of 60-90 N. They disintegrate completely in water at room temperature within 30-45 s.

## Example 2

The components of the previous Example, with the exception of water and magnesium stearate, are mixed for 20 minutes. Magnesium stearate (2 g) is added and mixing is continued for an additional 2 minutes. The resulting mixture is compressed into tablets of the type as mentioned in the previous Example. The resulting tablets have the same pharmaceutical properties as the tablets of the previous Example.

## Example 3

Following the same procedure as described in Example 1, 111 g of oxytetracycline dihydrate are granulated with 80 ml of water. Tablets each of 250 mg are provided by compression in the usual way. The resulting tablets have a disintegration time of 30 s and a hardness of 80 N.

## Example 4

Doxycycline monohydrate (11 g), microcrystalline cellulose (4.5 g), low-substituted hydroxypropyl cellulose (2.5 g), hydroxypropyl methylcellulose of 5 mPa·s (cP) viscosity (0.75 g), saccharin (1 g), maize starch (0.075 g), lactose (4.16 g) and colloidal silica (0.188 g) are mixed together during 20 minutes. Magnesium stearate (0.15 g) is added and mixing continued for an additional 2 minutes. The obtained mixture is compressed into tablets of about 250 mg with a diameter of about 9 mm and a hardness of 70-100 N. The tablets disintegrate completely in water of room temperature within 45-60 s.

## Example 5

The procedure of the previous experiment is repeated except that 4.5 g of microfine cellulose are used instead of 4.5 g of microcrystalline cellulose.

The resulting tablets have a hardness of 90-110 N and disintegrate in water of room temperature within 100 s.

## Claims

## Claims for the following Contracting States : AT, BE, CH, DE, FR, GB, IT, LI, LU, NL, SE

1. Pharmaceutical tablet comprising
a. a tetracycline,
b. either microcrystalline cellulose or microfine cellulose or a mixture of them in an amount of 20-60 wt%, preferably 35-50 wt% with respect to the tetracycline,
c. low-substituted hydroxypropylcellulose and a thickening agent with a viscosity of less than 50 mPa · s (cP) when measured as a 2 wt% aqueous solution at 20°C, in a weight ratio of 3 : 1 to 10 : 1, preferably 5 : 1, and which together amount to 10-40 wt%, preferably 20-30 wt%, with respect to the tetracycline.

2. Tablet according to claim 1, characterized in that the viscosity of the thickening agent in a 2 wt% solution at 20°C is less than 10 mPa · s (cP), preferably 5 mPa · s (cP).

3. Tablet according to claim 1 or 2, characterized in that the thickening agent is hydroxypropylmethylcellulose.

4. Pharmaceutical granulate comprising a tetracycline and either microcrystalline cellulose or microfine cellulose or a mixture of them in an amount of 20-60 wt%, preferably 35-50 wt%, with respect to the tetracycline and 0-0.5 wt%, preferably 0-0.1 wt%, of a wet granulation binding substance.

5. Process for the preparation by wet granulation of a pharmaceutical tablet containing
a. a tetracycline,
b. either microcrystalline cellulose or microfine cellulose or a mixture of them in an amount of 20-60 wt%, preferably 35-50 wt% with respect to the tetracycline,
c. low-substituted hydroxypropylcellulose and a thickening agent with a viscosity of less than 50 mPa · s (cP) when measured as a 2 wt% aqueous solution at 20°C, in a weight ratio of 3 : 1 to 10 : 1, preferably 5 : 1, and which together amount to 10-40 wt%, preferably 20-30 wt%, with respect to the tetracycline, which comprises :
(1) preparing a wet mass of the tetracycline with either microcrystalline cellulose or microfine cellulose or a mixture of them in said amounts, and water,
(2) granulating the wet mass,
(3) mixing the granulate with at least low-substituted hydroxypropylcellulose and said thickening agent in the mentioned amounts to an intimate mixture and
(4) compressing the resulting mixture to tablets.

6. Process for the preparation of a pharmaceutical tablet by direct compression, which comprises making an intimate mixture of
a. a tetracycline,
b. either microcrystalline cellulose or microfine cellulose or a mixture of them in an amount of 20-60 wt%, preferably 35-50 wt% with respect to the tetracycline,
c. low-substituted hydroxypropylcellulose and a thickening agent with a viscosity of less than 50 mPa · s (cP) when measured as a 2 wt% aqueous solution at 20°C, in a weight ratio of 3 : 1 to 10 :

1, preferably 5 : 1, and which together amount to 10-40 wt%, preferably 20-30 wt%, with respect to the tetracycline, and compressing the obtained mixture to tablets by methods known in the art.

7. Process according to claims 5 or 6, characterized in that the thickening agent in a 2 wt% solution at 20°C has a viscosity of less than 10 mPa · s (cP) preferably of 5 mPa · s (cP).

8. Process according to any one of claims 5-7, characterized in that the thickening agent is hydroxypropylmethylcellulose.

9. Process according to claim 5, characterized in that 40-80 wt%, preferably 50-70 wt% of water are used.

10. Process according to claim 5 or 9, characterized in that the wet mass is admixed with 0-0.5 wt%, preferably 0-0.1 wt% of a wet granulation binding substance.

11. Process for the preparation of a granulate suited for the process of claim 5 which comprises mixing

a. a tetracycline, either microcrystalline cellulose or microfine cellulose or a mixture of them in an amount of 20-60 wt%, preferably 35-50 wt%, with respect to the tetracycline, and

b. 0-0.5 wt%, preferably 0-0.1 wt% of a wet binding substance and water to a wet mass and granulating the mixture by methods known in the art, to give a granulate containing 0-0.5 wt%, preferably 0-0.1 wt% of a wet binding substance.

**Claims for the following Contracting States : ES, GR**

1. Process for the preparation of a pharmacentical tablet, the tablet comprising

a. a tetracycline,

b. either microcrystalline cellulose or microfine cellulose or a mixture of them in an amount of 20-60 wt%, preferably 35-50 wt% with respect to the tetracycline,

c. low-substituted hydroxypropylcellulose and a thickening agent with a viscosity of less than 50 mPa · s (cP) when measured as a 2 wt% aqueous solution at 20°C, in a weight ratio of 3 : 1 to 10 : 1, preferably 5 : 1, and which together amount to 10-40 wt%, preferably 20-30 wt%, with respect to the tetracycline.

2. Process according to claim 1, characterized in that the viscosity of the thickening agent in a 2 wt% solution at 20°C is less than 10 mPa · s (cP), preferably 5 mPa · s (cP).

3. Process according to claim 1 or 2, characterized in that the thickening agent is hydroxypropylmethylcellulose.

4. Process for the preparation of a pharmaceutical granulate, the granulate comprising a tetracycline and either microcrystalline cellulose or microfine cellulose or a mixture of them in an amount of 20-60 wt%, preferably 35-50 wt%, with respect to the tetracycline and 0-0.5 wt%, preferably 0-0.1 wt%, of a wet granulation binding substance.

5. Process for the preparation by wet granulation of a pharmaceutical tablet containing

a. a tetracycline,

b. either microcrystalline cellulose or microfine cellulose or a mixture of them in an amount of 20-60 wt%, preferably 35-50 wt% with respect to the tetracycline,

c. low-substituted hydroxypropylcellulose and a thickening agent with a viscosity of less than 50 mPa · s (cP) when measured as a 2 wt% aqueous solution at 20°C, in a weight ratio of 3 : 1 to 10 : 1, preferably 5 : 1, and which together amount to 10-40 wt%, preferably 20-30 wt%, with respect to the tetracycline, which comprises :

(1) preparing a wet mass of the tetracycline with either microcrystalline cellulose or microfine cellulose or a mixture of them in said amounts, and water,

(2) granulating the wet mass,

(3) mixing the granulate with at least low-substituted hydroxypropylcellulose and said thickening agent in the mentioned amounts to an intimate mixture and

(4) compressing the resulting mixture to tablets.

6. Process for the preparation of a pharmaceutical tablet by direct compression, which comprises making an intimate mixture of

a. a tetracycline,

b. either microcrystalline cellulose or microfine cellulose or a mixture of them in an amount of 20-60 wt%, preferably 35-50 wt% with respect to the tetracycline,

c. low-substituted hydroxypropylcellulose and a thickening agent with a viscosity of less than 50 mPa · s (cP) when measured as a 2 wt% aqueous solution at 20°C, in a weight ratio of 3 : 1 to 10 : 1, preferably 5 : 1, and which together amount to 10-40 wt%, preferably 20-30 wt%, with respect to the tetracycline, and compressing the obtained mixture to tablets by methods known in the art.

7. Process according to claims 5 or 6, characterized in that the thickening agent in a 2 wt% solution at 20°C has a viscosity of less than 10 mPa · s (cP) preferably of 5 mPa · s (cP).

8. Process according to any one of claims 5-7, characterized in that the thickening agent is hydroxypropylmethylcellulose.

9. Process according to claim 5, characterized in that 40-80 wt%, preferably 50-70 wt% of water are used.

10. Process according to claim 5 or 9, characterized in that the wet mass is admixed with 0-0.5 wt%, preferably 0-0.1 wt% of a wet granulation binding substance.

11. Process for the preparation of a granulate suited for the process of claim 5 which comprises mixing

    a. a tetracycline, either microcrystalline cellulose or microfine cellulose or a mixture of them in an amount of 20-60 wt%, preferably 35-50 wt%, with respect to the tetracycline, and

    b. 0-0.5 wt%, preferably 0-0.1 wt% of a wet granulation binding substance and water to a wet mass and granulating the mixture by methods known in the art, to give a granulate containing 0-0.5 wt%, preferably 0-0.1 wt% of a wet granulation binding substance.

## Patentansprüche

### Patentansprüche für folgende Vertragsstaaten : AT, BE, CH, DE, FR, GB, IT, LI, LU, NL, SE

1. Pharmazeutische Tablette, die aufweist :
a. ein Tetracyclin,
b. entweder mikrokristalline Cellulose oder mikrofeine Cellulose oder ein Gemisch von diesen in einer Menge von 20 bis 60 Gew.-%, vorzugsweise 35 bis 50 Gew.-%, bezogen auf das Tetracyclin,
c. niedrigsubstituierte Hydroxypropylcellulose und ein Verdickungsmittel mit einer Viskosität von weniger als 50 mPa · s (cP), gemessen in Form einer 2-gewichtsprozentigen wässrigen Lösung bei 20°C, in einem Gewichtsverhältnis von 3 : 1 bis 10 : 1, vorzugsweise 5 : 1, und die zusammen 10 bis 40 Gew.-%, vorzugsweise 20 bis 30 Gew.-%, bezogen auf das Tetracyclin, betragen.

2. Tablette nach Anspruch 1, dadurch gekennzeichnet, dass die Viskosität des Verdickungsmittels in einer 2-gewichtsprozentigen Lösung bei 20°C geringer als 10 mPa · s (cP), vorzugsweise 5 mPa · s (cP), ist.

3. Tablette nach Anspruch 1 oder 2, dadurch gekennzeichnet, dass das Verdickungsmittel Hydroxypropylmethylcellulose ist.

4. Pharmazeutisches Granulat, das ein Tetracyclin und entweder mikrokristalline Cellulose oder mikrofeine Cellulose oder ein Gemisch von diesen in einer Menge von 20 bis 60 Gew.-%, vorzugsweise 35 bis 50 Gew.-%, bezogen auf das Tetracyclin, sowie 0 bis 0,5 Gew.-%, vorzugsweise 0 bis 0,1 Gew.-%, einer Feuchtgranulierungsbindesubstanz aufweist.

5. Verfahren zur Herstellung durch Feuchtgranulierung einer pharmazeutischen Tablette, die enthält:
a. ein Tetracyclin,
b. entweder mikrokristalline Cellulose oder mikrofeine Cellulose oder ein Gemisch von diesen in einer Menge von 20 bis 60 Gew.-%, vorzugsweise 35 bis 50 Gew.-%, bezogen auf das Tetracyclin,
c. niedrigsubstituierte Hydroxypropylcellulose und ein Verdickungsmittel mit einer Viskosität von weniger als 50 mPa · s (cP), gemessen in Form einer 2-gewichtsprozentigen wässrigen Lösung bei 20°C, in einem Gewichtsverhältnis von 3 : 1 bis 10 : 1, vorzugsweise 5 : 1, und die zusammen 10 bis 40 Gew.-%, vorzugsweise 20 bis 30 Gew.-%, bezogen auf das Tetracyclin, betragen, dadurch gekennzeichnet, dass man

    (1) eine feuchte Masse des Tetracyclins mit entweder mikrokristalliner Cellulose oder mikrofeiner Cellulose oder einem Gemisch von diesen in den genannten Mengen und Wasser herstellt,
    (2) die feuchte Masse granuliert,
    (3) das Granulat mit mindestens niedrigsubstituierter Hydroxypropylcellulose und dem genannten Verdickungsmittel in den erwähnten Mengen zu einem innigen Gemisch mischt und
    (4) das resultierende Gemisch zu Tabletten verpresst.

6. Verfahren zur Herstellung einer pharmazeutischen Tablette durch Direktverpressen, dadurch gekennzeichnet, dass man ein inniges Gemisch aus
a. einem Tetracyclin,
b. entweder mikrokristalliner Cellulose oder mikrofeiner Cellulose oder einem Gemisch von diesen in einer Menge von 20 bis 60 Gew.-%, vorzugsweise 35 bis 50 Gew.-%, bezogen auf das Tetracyclin,
c. niedrigsubstituierter Hydroxypropylcellulose und einem Verdickungsmittel mit einer Viskosität von weniger als 50 mPa · s (cP), gemessen in Form einer 2-gewichtsprozentigen wässrigen Lösung bei 20°C, in einem Gewichtsverhältnis von 3 : 1 bis 10 : 1, vorzugsweise 5 : 1, und die zusammen 10 bis 40 Gew.-%, vorzugsweise 20 bis 30 Gew.-%, bezogen auf das Tetracyclin, betragen, herstellt und das erhaltene Gemisch mittels dem Fachmann bekannter Methoden zu Tabletten verpresst.

7. Verfahren nach Anspruch 5 oder 6, dadurch gekennzeichnet, dass das Verdickungsmittel in Form einer 2-gewichtsprozentigen Lösung bei 20°C eine Viskosität von weniger als 10 mPa · s (cP), vorzugsweise von 5 mPa · s (cP), hat.

8. Verfahren nach einem der Ansprüche 5 bis 7, dadurch gekennzeichnet, dass das Verdickungsmittel Hydroxypropylmethylcellulose ist.

9. Verfahren nach Anspruch 5, dadurch gekennzeichnet, dass 40 bis 80 Gew.-%, vorzugsweise 50 bis 70 Gew.-%, Wasser verwendet werden.

10. Verfahren nach Anspruch 5 oder 9, dadurch gekennzeichnet, dass die feuchte Masse mit 0 bis 0,5 Gew.-%, vorzugsweise 0 bis 0,1 Gew.-%, einer Feuchtgranulierungsbindesubstanz vermischt wird.

11. Verfahren zur Herstellung eines Granulates, das sich für das Verfahren von Anspruch 5 eignet, dadurch gekennzeichnet, dass man

a. ein Tetracyclin, entweder mikrokristalline Cellulose oder mikrofeine Cellulose oder ein Gemisch von diesen in einer Menge von 20 bis 60 Gew.-%, vorzugsweise 35 bis 50 Gew.-%, bezogen auf das Tetracyclin, und

b. 0 bis 0,5 Gew.-%, vorzugsweise 0 bis 0,1 Gew.-%, einer Feuchtgranulierungsbindesubstanz und Wasser zu einer feuchten Masse mischt und das Gemisch mittels dem Fachmann bekannter Methoden granuliert unter Bildung eines Granulates, das 0 bis 0,5 Gew.-%, vorzugsweise 0 bis 0,1 Gew.-%, einer Feuchtgranulierungsbindesubstanz enthält.

**Patentansprüche für folgende Vertragsstaaten : ES, GR**

1. Verfahren zur Herstellung einer pharmazeutischer Tablette, die aufweist :

a. ein Tetracyclin,

b. entweder mikrokristalline Cellulose oder mikrofeine Cellulose oder ein Gemisch von diesen in einer Menge von 20 bis 60 Gew.-%, vorzugsweise 35 bis 50 Gew.-%, bezogen auf das Tetracyclin,

c. niedrigsubstituierte Hydroxypropylcellulose und ein Verdickungsmittel mit einer Viskosität von weniger als 50 mPa · s (cP), gemessen in Form einer 2-gewichtsprozentigen wässrigen Lösung bei 20°C, in einem Gewichtsverhältnis von 3 : 1 bis 10 : 1, vorzugsweise 5 : 1, und die zusammen 10 bis 40 Gew.-%, vorzugsweise 20 bis 30 Gew.-%, bezogen auf das Tetracyclin, betragen.

2. Verfahren nach Anspruch 1, dadurch gekennzeichnet, dass die Viskosität des Verdickungsmittels in einer 2-gewichtsprozentigen Lösung bei 20°C geringer als 10 mPa · s (cP), vorzugsweise 5 mPa · s (cP), ist.

3. Verfahren nach Anspruch 1 oder 2, dadurch gekennzeichnet, dass das Verdickungsmittel Hydroxypropylmethylcellulose ist.

4. Verfahren zur Herstellung eines pharmazeutischen Granulates, das ein Tetracyclin und entweder mikrokristalline Cellulose oder mikrofeine Cellulose oder ein Gemisch von diesen in einer Menge von 20 bis 60 Gew.-%, vorzugsweise 35 bis 50 Gew.-%, bezogen auf das Tetracyclin, sowie 0 bis 0,5 Gew.-%, vorzugsweise 0 bis 0,1 Gew.-%, einer Feuchtgranulierungsbindesubstanz aufweist.

5. Verfahren zur Herstellung durch Feuchtgranulierung einer pharmazeutischen Tablette, die enthält:

a. ein Tetracyclin,

b. entweder mikrokristalline Cellulose oder mikrofeine Cellulose oder ein Gemisch von diesen in einer Menge von 20 bis 60 Gew.-%, vorzugsweise 35 bis 50 Gew.-%, bezogen auf das Tetracyclin,

c. niedrigsubstituierte Hydroxypropylcellulose und ein Verdickungsmittel mit einer Viskosität von weniger als 50 mPa · s (cP), gemessen in Form einer 2-gewichtsprozentigen wässrigen Lösung bei 20°C, in einem Gewichtsverhältnis von 3 : 1 bis 10 : 1, vorzugsweise 5 : 1, und die zusammen 10 bis 40 Gew.-%, vorzugsweise 20 bis 30 Gew.-%, bezogen auf das Tetracyclin, betragen, dadurch gekennzeichnet, dass man

(1) eine feuchte Masse des Tetracyclins mit entweder mikrokristalliner Cellulose oder mikrofeiner Cellulose oder einem Gemisch von diesen in den genannten Mengen und Wasser herstellt,

(2) die feuchte Masse granuliert,

(3) das Granulat mit mindestens niedrigsubstituierter Hydroxypropylcellulose und den genannten Verdickungsmittel in den erwähnten Mengen zu einem innigen Gemisch mischt und

(4) das resultierende Gemisch zu Tabletten verpresst.

6. Verfahren zur Herstellung einer pharmazeutischen Tablette durch Direktverpressen, dadurch gekennzeichnet, dass man ein inniges Gemisch aus

a. einem Tetracyclin,

b. entweder mikrokristalliner Cellulose oder mikrofeiner Cellulose oder einem Gemisch von diesen in einer Menge von 20 bis 60 Gew.-%, vorzugsweise 35 bis 50 Gew.-%, bezogen auf das Tetracyclin,

c. niedrigsubstituierter Hydroxypropylcellulose und einem Verdickungsmittel mit einer Viskosität von weniger als 50 mPa · s (cP), gemessen in Form einer 2-gewichtsprozentigen wässrigen Lösung bei 20°C, in einem Gewichtsverhältnis von 3 : 1 bis 10 : 1, vorzugsweise 5 : 1, und die zusammen 10 bis 40 Gew.-%, vorzugsweise 20 bis 30 Gew.-%, bezogen auf das Tetracyclin, betragen, herstellt und das erhaltene Gemisch mittels dem Fachmann bekannter Methoden zu Tabletten verpresst.

7. Verfahren nach Anspruch 5 oder 6, dadurch gekennzeichnet, dass das Verdickungsmittel in Form einer 2-gewichtsprozentigen Lösung bei 20°C eine Viskosität von weniger als 10 mPa · s (cP), vorzugsweise von 5 mPa · s (cP), hat.

8. Verfahren nach einem der Ansprüche 5 bis 7, dadurch gekennzeichnet, dass das Verdickungsmittel Hydroxypropylmethylcellulose ist.

9. Verfahren nach Anspruch 5, dadurch gekennzeichnet, dass 40 bis 80 Gew.-%, vorzugsweise 50 bis 70 Gew.-%, Wasser verwendet werden.

10. Verfahren nach Anspruch 5 oder 9, dadurch gekennzeichnet, dass die feuchte Masse mit 0 bis 0,5 Gew.-%, vorzugsweise 0 bis 0,1 Gew.-%, einer Feuchtgranulierungsbindesubstanz vermischt wird.

11. Verfahren zur Herstellung eines Granulates, das sich für das Verfahren von Anspruch 5 eignet, dadurch gekennzeichnet, dass man

a. ein Tetracyclin, entweder mikrokristalline Cellulose oder mikrofeine Cellulose oder ein Gemisch von diesen in einer Menge von 20 bis 60 Gew.-%, vorzugsweise 35 bis 50 Gew.-%, bezogen auf das Tetracyclin, und

b. 0 bis 0,5 Gew.-%, vorzugsweise 0 bis 0,1 Gew.-%, einer Feuchtgranulierungsbindesubstanz und Wasser zu einer feuchten Masse mischt und das Gemisch mittels dem Fachmann bekannter Methoden granuliert unter Bildung eines Granulates, das 0 bis 0,5 Gew.-%, vorzugsweise 0 bis 0,1 Gew.-%, einer Feuchtgranulierungsbindesubstanz enthält.

## Revendications

### Revendications pour les Etats contractants suivants : AT, BE, CH, DE, FR, GB, IT, LI, LU, NL, SE

1. Comprimé pharmaceutique comprenant
a. une tétracycline,
b. de la cellulose microcristalline ou de la cellulose microfine, ou bien un mélange de celles-ci, en une quantité de 20 à 60% en poids, de préférence de 35 à 50% en poids, par rapport à la tétracycline,
c. de l'hydroxypropylcellulose faiblement substituée et un épaississant ayant une viscosité inférieure à 50 mPa · s (cP), telle que mesurée sur une solution aqueuse à 2% en poids à 20°C, dans un rapport pondéral de 3 : 1 à 10 : 1 et de préférence de 5 : 1, qui font ensemble 10 à 40% en poids et de préférence 20 à 30% en poids, par rapport à la tétracycline.

2. Comprimé suivant la revendication 1, caractérisé en ce que la viscosité de l'épaississant en solution à 2% en poids à 20°C est inférieure à 10 mPa · s (cP), de préférence de 5 mPa · s (cP).

3. Comprimé suivant la revendication 1 ou 2, caractérisé en ce que l'épaississant est l'hydroxypropylméthylcellulose.

4. Granules pharmaceutiques comprenant une tétracycline et une cellulose microcristalline ou une cellulose microfine, ou bien un mélange de celles-ci, en une quantité de 20 à 60% en poids, de préférence de 35 à 50% en poids, par rapport à la tétracycline, et 0 à 0,5% en poids, de préférence 0 à 0,1% en poids, d'un liant pour voie humide.

5. Procédé de préparation par granulation au mouillé d'un comprimé pharmaceutique contenant

a. une tétracycline,
b. de la cellulose microcristalline ou de la cellulose microfine, ou bien un mélange de celles-ci, en une quantité de 20 à 60% en poids, de préférence de 35 à 50% en poids, par rapport à la tétracycline,
c. de l'hydroxypropylcellulose faiblement substituée et un épaississant ayant une viscosité inférieure à 50 mPa · s (cP), telle que mesurée sur une solution aqueuse à 2% en poids à 20°C, dans un rapport pondéral de 3 : 1 à 10 : 1 et de préférence de 5 : 1, qui font ensemble 10 à 40% en poids et de préférence 20 à 30% en poids, par rapport à la tétracycline, qui comprend :

(1) la préparation d'une masse humide de la tétracycline avec la cellulose microcristalline ou la cellulose microfine, ou bien un mélange de celles-ci, en ces quantités, et de l'eau,
(2) la granulation de la masse humide,
(3) le mélange des granules avec au moins l'hydroxypropylcellulose faiblement substituée et l'épaississant en les quantités mentionnées pour former un mélange intime, et
(4) le pressage du mélange résultant en comprimés.

6. Procédé de préparation d'un comprimé pharmaceutique par pressage direct, qui comprend la formation d'un mélange intime comprenant

a. une tétracycline,
b. de la cellulose microcristalline ou de la cellulose microfine, ou bien un mélange de celles-ci, en une quantité de 20 à 60% en poids, de préférence de 35 à 50% en poids, par rapport à la tétracycline,
c. de l'hydroxypropylcellulose faiblement substituée et un épaississant ayant une viscosité inférieure à 50 mPa · s (cP), telle que mesurée sur une solution aqueuse à 2% en poids à 20°C, dans un rapport pondéral de 3 : 1 à 10 : 1 et de préférence de 5 : 1, qui font ensemble 10 à 40% en poids et de préférence 20 à 30% en poids, par rapport à la tétracycline, et le pressage du mélange résultant en comprimés suivant des procédés connus.

7. Procédé suivant la revendication 5 ou 6, caractérisé en ce que l'épaississant, en solution à 2% en poids à 20°C, a une viscosité inférieure à 10 mPa · s (cP), de préférence de 5 mPa · s (cP).

8. Procédé suivant l'une quelconque des revendications 5 à 7, caractérisé en ce que l'épaississant est l'hydroxypropylméthylcellulose.

9. Procédé suivant la revendication 5, caractérisé en ce qu'on utilise 40 à 80% en poids et de préférence 50 à 70% en poids d'eau.

10. Procédé suivant la revendication 5 ou 9, caractérisé en ce que la masse humide est mélangée avec 0 à 0,5% en poids, de préférence 0 à 0,1% en poids, d'un liant pour voie humide.

11. Procédé de préparation de granules convenant pour le procédé suivant la revendication 5, qui comprend le mélange

a. d'une tétracycline, d'une cellulose microcristalline ou d'une cellulose microfine, ou bien d'un mélange de celles-ci, en une quantité de 20 à 60% en poids, de préférence de 35 à 50% en poids, par rapport à la tétracycline, et

b. de 0 à 0,5% en poids, de préférence 0 à 0,1% en poids, d'un liant pour voie humide et d'eau en une masse humide et la granulation du mélange suivant des procédés connus pour former des granules contenant 0 à 0,5% en poids, de préférence 0 à 0,1% en poids d'un liant pour voie humide.

**Revendications pour les Etats contractants suivants : ES, GR**

1. Procédé de préparation d'un comprimé pharmaceutique, le comprimé comprenant :

a. une tétracycline,

b. de la cellulose microcristalline ou de la cellulose microfine, ou bien un mélange de celles-ci, en une quantité de 20 à 60% en poids, de préférence de 35 à 50% en poids, par rapport à la tétracycline,

c. de l'hydroxypropylcellulose faiblement substituée et un épaississant ayant une viscosité inférieure à 50 mPa · s (cP), telle que mesurée sur une solution aqueuse à 2% en poids à 20°C, dans un rapport pondéral de 3 : 1 à 10 : 1 et de préférence de 5 : 1, qui font ensemble 10 à 40% en poids et de préférence 20 à 30% en poids, par rapport à la tétracycline.

2. Procédé suivant la revendication 1, caractérisé en ce que la viscosité de l'épaississant en solution à 2% en poids à 20°C est inférieure à 10 mPa · s (cP), de préférence de 5 mPa · s (cP).

3. Procédé suivant la revendication 1 ou 2, caractérisé en ce que l'épaississant est l'hydroxypropylméthylcellulose.

4. Procédé de préparation de granules pharmaceutiques, les granules comprenant une tétracycline et une cellulose microcristalline ou une cellulose microfine, ou bien un mélange de celles-ci, en une quantité de 20 à 60% en poids, de préférence de 35 à 50% en poids, par rapport à la tétracycline, et 0 à 0,5% en poids, de préférence 0 à 0,1% en poids, d'un liant pour voie humide.

5. Procédé de préparation par granulation au mouillé d'un comprimé pharmaceutique contenant

a. une tétracycline,

b. de la cellulose microcristalline ou de la cellulose microfine, ou bien un mélange de celles-ci, en une quantité de 20 à 60% en poids, de préférence de 35 à 50% en poids, par rapport à la tétracycline,

c. de l'hydroxypropylcellulose faiblement substituée et un épaississant ayant une viscosité inférieure à 50 mPa · s (cP), telle que mesurée sur une solution aqueuse à 2% en poids a 20°C, dans un rapport pondéral de 3 : 1 à 10 : 1 et de préférence 5 : 1, qui font ensemble 10 à 40% en poids et de préférence 20 à 30% en poids, par rapport à la tétracycline, qui comprend :

(1) la préparation d'une masse humide de la tétracycline avec la cellulose microcristalline ou de la cellulose microfine, ou bien un mélange de celles-ci, en ces quantités, et de l'eau,

(2) la granulation de la masse humide,

(3) le mélange des granules avec au moins l'hydroxypropylcellulose faiblement substituée et l'épaississant en les quantités mentionnées pour former un mélange intime, et

(4) le pressage du mélange résultant en comprimés.

6. Procédé de préparation d'un comprimé pharmaceutique par pressage direct, qui comprend la formation d'un mélange intime comprenant

a. une tétracycline,

b. de la cellulose microcristalline ou de la cellulose microfine, ou bien un mélange de celles-ci, en une quantité de 20 à 60% en poids, de préférence de 35 à 50% en poids, par rapport à la tétracycline,

c. de l'hydroxypropylcellulose faiblement substituée et un épaississant ayant une viscosité inférieure à 50 mPa · s (cP), telle que mesurée sur une solution aqueuse à 2% en poids à 20°C, dans un rapport pondéral de 3 : 1 à 10 : 1 et de préférence de 5 : 1, qui font ensemble 10 à 40% en poids et de préférence 20 à 30% en poids, par rapport à la tétracycline, et le pressage du mélange résulant en comprimés suivant des procédés connus.

7. Procédé suivant la revendication 5 ou 6, caractérisé en ce que l'épaississant, en solution à 2% en poids à 20°C, a une viscosité inférieure à 10 mPa · s (cP), de préférence de 5 mPa · s (cP).

8. Procédé suivant l'une quelconque des revendications 5 a 7, caractérisé en ce que l'épaississant est l'hydroxypropylméthylcellulose.

9. Procédé suivant la revendication 5, caractérisé en ce qu'on utilise 40 à 80% en poids et de préférence 50 à 70% en poids d'eau.

10. Procédé suivant la revendication 5 ou 9, caractérisé en ce que la masse humide est mélangée avec 0 à 0,5% en poids, de préférence 0 à 0,1% en poids, d'un liant pour voie humide.

11. Procédé de préparation de granules convenant pour le procédé suivant la revendication 5, qui comprend le mélange

a. d'une tétracycline, d'une cellulose microcristalline ou d'une cellulose microfine, ou bien d'un mélange de celles-ci, en une quantité de 20 à 60% en poids, de préférence de 35 à 50% en poids, par rapport à la tétracycline, et

b. de 0 à 0,5% en poids, de préférence 0 à 0,1% en poids, d'un liant pour voie humide et d'eau en une masse humide et la granulation du mélange suivant des procédés connus pour former des granules contenant 0 à 0,5% en poids, de préférence 0 à 0,1% en poids, d'un liant pour voie humide.